# EUROPÄISCHE PATENTSCHRIFT

(11) **EP 1 912 606 B1**
(45) Veröffentlichungstag und Bekanntmachung des Hinweises auf die Patenterteilung: **22.07.2015**
(21) Anmeldenummer: 06776571.9
(22) Anmeldetag: 02.08.2006
(51) Int. Cl.: A61F 13/15, A61F 13/20

(54) **VERFAHREN ZUM HERSTELLEN EINES IN EINER SCHUTZHÜLLE KONFEKTIONIERTEN TAMPONS**
METHOD FOR PRODUCING A TAMPON WRAPPED IN A PROTECTIVE COVER
PROCEDE DE FABRICATION D'UN TAMPON FABRIQUE DANS UNE GAINE DE PROTECTION

(30) Priorität: 05.08.2005 DE 102005037065
(43) Veröffentlichungstag der Anmeldung: 23.04.2008
(73) Patentinhaber: Ruggli Projects AG, 6332 Hagendorn (CH)
(72) Erfinder: ROLLI, Kilian, CH-5400 Baden (CH)
(74) Vertreter: Burger, Hannes
(86) Internationale Anmeldenummer: PCT/EP2006/007659
(87) Internationale Veröffentlichungsnummer: WO 2007/017173

(56) Entgegenhaltungen:
- WO-A-2004/100847
- WO-A2-03/007862
- DE-A1- 1 815 541

## Beschreibung

Die Erfindung betrifft ein Verfahren zum Herstellen eines in einer Schutzhülle konfektionierten Tampons aus einem vorzugsweise durch Aufwickeln eines Faservliesstreifens gebildeten Vorformling, welcher in getrennten Verformungsstufen durch von aussen auf den Vorformling einwirkende Druckkräfte zu dem Tampon verformt, und welcher zur Einzelkonfektionierung mit einer den Tampon fest umschliessenden Folienhülse als Schutzhülle versehen wird.

Ein Verfahren zur Herstellung von Tampons ist aus der WO 2004/100847 bekannt. Die dort beschriebenen Tampons werden unter Zufuhr von Gas bzw. Feuchtigkeit stabilisiert. Sie können optional auch mit einer Schutzhülle (overwrap), bestehend aus Rayon, Baumwolle, verschiedenen Fasern und Mischungen davon bzw. Vliesstoffen gebildet sein. Diese Hüllen bzw. Overwraps sind vorbehandelt, sodass sie feuchtigkeitsleitend, feuchtigkeitshemmend sein können bzw. eine Dochtwirkung oder keine Dochtwirkung ausüben können und können auch eine wasserabweisende Wirkung aufweisen. Derartige Hüllen bzw. Overwraps dienen dazu, dass Ausfasern der Tampons beim Entnehmen der mit Feuchtigkeit gefüllten Tampons aus dem Körper an deren Oberfläche zu verhindern. Derartige Tampons können auch mit einer weiteren Hülle versehen sein. Nachteilig ist hierbei, dass zur Herstellung von nicht-runden Tampons ein hoher apparativer Aufwand erforderlich ist.

Ein weit verbreitetes Verfahren zur Herstellung von Tampons (Menstruationstampons) ist aus der DE 39 34 153 C2 bekannt. Das Pressen des aus einem aufgewickelten Faservliesstreifen bestehenden Vorformlings erfolgt hierbei mehrstufig. In einer ersten Stufe erfolgt die

Formung durch zwei Gruppen von zentripetal zur Längsachse zustellbaren Pressbacken, welche mit Pressschneiden versehen sind, um so den Vorformling zu einem Rillentampon mit Längsnuten und dazwischen angeordneten Rippen zu formen. Mittels eines Stössels wird der Vorformling aus den in geschlossener Stellung verharrenden oder geringfügig öffnenden Pressbacken axial herausgestossen und durch ein sich anschliessendes Formwerkzeug gepresst. Dieses Formwerkzeug ist konisch gestaltet, mit einem dem Aussendurchmesser des Vorformlings entsprechenden Eintrittsdurchmesser, und einem dem endgültigen Aussendurchmesser des Tampons entsprechenden Austrittsdurchmessers.

Zu Verpackungs und Hygienezwecken wird der Tampon anschliessend mit einer den Tampon fest umschliessenden Schutzhülle versehen, d.h. konfektioniert. Die Schutzhülle wird getrennt aus einer Folienhülse gebildet. Beim Einsetzen des Tampons in die Folienhülse erleichtert die im Wesentlichen zylindrische Gestalt des Tampons das axiale Hineinführen in die ebenfalls zylindrische Folienhülse. Schliesslich ist die Folienhülse zur Vervollständigung der Schutzhülle zu verschliessen, wozu geeignete Verfahren z.B. in der DE 19 55 600 C1 und der EP 1 010 622 A1 beschrieben sind.

Mit den genannten Verfahren lassen sich nahezu alle Tampons herstellen und fertig konfektionieren, deren Querschnitt im Wesentlichen kreisförmig ist. Es sind jedoch auch bereits, so z. B. in der DE 18 15 374 A, Tampons mit von der Kreisform abweichender Querschnittsfläche vorgeschlagen worden, z.B. Tampons mit leicht elliptischen oder ovalen Querschnitten. Das Hineinführen solcher Tampons in eine vorgefertigte Folienhülse erfordert einen erhöhten apparativen Aufwand.

Der Erfindung liegt die **Aufgabe** zugrunde, den Produktionsaufwand für die Herstellung eines Tampons und insbesondere eines nicht runden Tampons bis zu dessen Einzelkonfektionierung in einer Schutzhülle zu verringern.

Zur **Lösung** dieser Aufgabe wird bei einem Verfahren mit den eingangs angegebenen Merkmalen vorgeschlagen, dass
- die Folienhülse (10) als eine für Feuchtigkeit undurchlässige, unperforierte und zylindrisch glatt vorgeformte, zumindest die Mantelfläche des Vorformlings (V) umschließende Hülse hergestellt wird;
- danach der im Wesentlichen zylindrische Vorformling (V) noch vor der letzten Verformungsstufe (15) in die Folienhülse (10) eingeschoben wird, und
- dass der Vorformling (V) einschließlich der ihn umschließenden Folienhülse (10) anschließend der letzten Verformungsstufe (15) zugeführt und dort zwischen mindestens zwei relativ zueinander beweglichen Druckbacken (20a, 20b) bis zum Erreichen einer nicht zylindrischen Endgestalt verformt wird und
- der Tampon (T) durch dichtes Verschließen der Folienhülse (10) einzeln verpackt wird.

Der Erfindung liegt daher der Gedanke zugrunde, den Tampon bereits vor der Durchführung der letzten, d.h. abschliessenden Verformung mit der Folienhülse zur dichten Einzelverpackung des Tampons zu versehen. Erst danach wird der Vorformling der letzten Verformungsstufe zugeführt. Diese abschliessende Druckbeaufschlagung erfolgt zwischen mindestens zwei relativ zueinander beweglichen Pressbacken und bis zum Erreichen der endgültigen, von einer zylindrischen Querschnittsform abweichende Gestalt des Tampons. Hierbei wird der Umstand ausgenutzt, dass bei einer vorgegebenen Querschnittsfläche der diese Fläche umgebende Umfang dann am geringsten ist, wenn die Fläche eine Kreisfläche ist. Umgekehrt führt die Überführung eines Kreisquerschnittes in eine andere Querschnittsform bei gleich bleibendem Umfang zu einer Querschnittsverringerung und, sofern ein Volumen umschlossen ist, zu einer Verdichtung des umgeschlossenen Materials. Die erfindungsgemäss vorgesehene letzte Pressstufe führt daher, ein hinreichend reckarmes Folienmaterial vorausgesetzt, in dem Tampon zu einer zusätzlichen Verdichtung des Vliesmaterials. Durch ein dichtes Verschließen des an die Außenform des verformten Tampons angepasste Folienhülse kann daher jeder Tampon einzeln verpackt werden.

Mit einer Ausgestaltung des Verfahrens wird vorgeschlagen, dass die beiden Enden der Folienhülse verschlossen werden, bevor der Vorformling einschliesslich der ihn vollständig umschliessenden Folienhülse der letzten Verformungsstufe zugeführt wird. Noch vorteilhafter ist jedoch eine Reihenfolge, bei der die beiden Enden der Folienhülse erst verschlossen werden, nachdem der Vorformling zwischen den Pressbacken zu seiner Endgestalt verformt wurde.

Für die Folienhülse wird ein sich möglichst reckarmes Verhalten des Folienmaterials angestrebt. Gemäß einer Ausgestaltung wird hierzu die Verwendung eines Folienmaterials auf Basis von Cellophan oder Polypropylen vorgeschlagen.

Weitere Einzelheiten und Vorteile ergeben sich aus der nachfolgenden Beschreibung eines Ausführungsbeispieles, welches auf der Zeichnung dargestellt ist. Darin zeigen im Einzelnen:
- Figur 1: eine schematische Darstellung der Hauptbestandteile einer Vorrichtung zum Pressen und Einzelverpacken von Tampons;
- Figur 2: einen vergrößerten Querschnitt entlang der in Figur 1 mit II-II bezeichneten Ebene einschließlich eines von einer Folienhülse umgebenden Tampon-Vorförmlings;
- Figur 3: den fertigen Tampon nach Durchlaufen der letzten Pressstufe und
- Figur 4: eine mathematische Herleitung der Verdichtungsverhältnisse im Tamponquerschnitt vor und nach der letzten Druckbeaufschlagung.

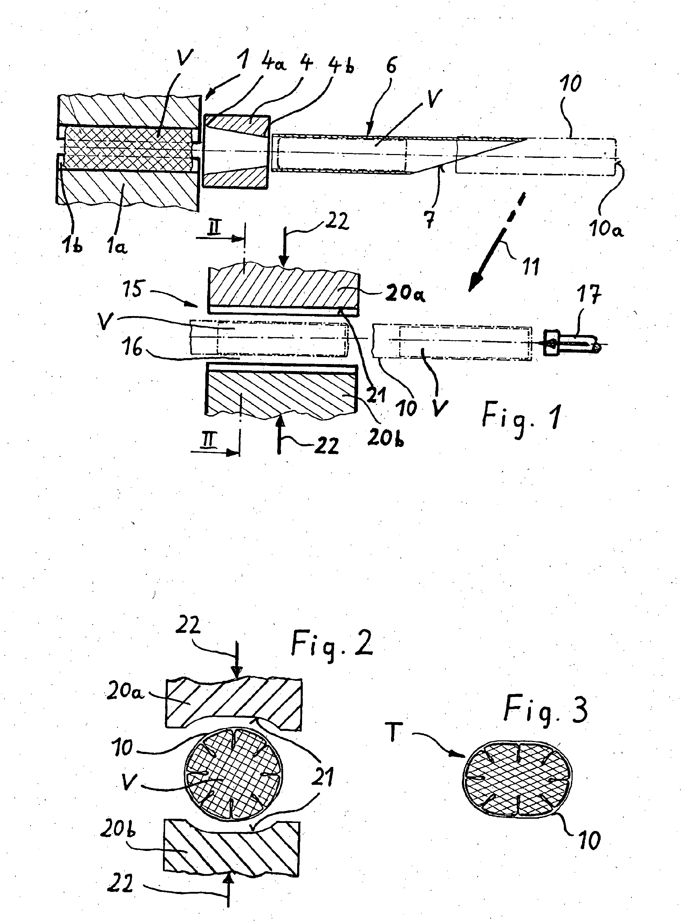

Mit der in Figur 1 vereinfacht dargestellten Vorrichtung lässt sich das erfindungsgemäße Verfahren durchführen. Mit dem Bezugszeichen 1 ist ein Presswerkzeug bezeichnet, welches sich beim Ausführungsbeispiel aus acht in radialer Richtung beweglichen Pressbacken 1a zusammensetzt. Die Pressbacken 1a sind mit Pressflächen versehen und weisen ferner jeweils eine in radialer Richtung nochmals weiter vorstehende Pressschneide 1b auf. Wenn die Pressbacken 1a des Presswerkzeuges nach dem Einführen eines aus saugfähigen Fasern bestehenden Rohlings beliebiger Querschnittsform geschlossen worden sind, ergibt sich ein Vorformling V mit einem Kern höherer Verdichtung und einer Randzone aus axial verlaufenden Rippen und zwischen den Rippen verlaufenden, das Absorptionsverhalten des späteren fertigen Tampons verbessernden Längsnuten.

Aus dem Presswerkzeug 1 wird der Vorformling V mittels eines auf der Zeichnung nicht dargestellten Ausschiebestößels axial ausgeschoben. In Ausschieberichtung hinter dem Presswerkzeug 1 ist ein Formwerkzeug 4 mit konisch zulaufender Innenkontur angeordnet. Der Eintrittsdurchmesser 4a des Formwerkzeuges 4 ist gleich oder geringfügig größer als der Außendurchmesser des Vorformlings V. Der Austrittsdurchmesser 4b des Formwerkzeuges 4 bestimmt den Außendurchmesser des aus dem Formwerkzeug 4 austretenden, und daher in seinem Durchmesser nochmals reduzierten Vorformlings. Das Formwerkzeug 4 stellt daher die zweite Verformungsstufe für den Vorformling V dar.

An den Austrittsdurchmesser 4b des Formwerkzeuges 4 schließt sich, wiederum in Ausschieberichtung des Ausschiebestößels, eine zwar dünnwandige aber starre Führungshülse 6 an, welche an ihrem anderen Ende in einer Schräge 7 ausläuft.

Von der anderen Seite her, d.h. in Figur 1 von rechts her, lässt sich auf die Schräge 7 der dünnwandigen Führungshülse 6 eine an anderer Stelle vorgefertigte zylindrische Folienhülse 10 axial aufschieben. Die Folienhülse 10 ist eine zylindrisch glatte, unperforierte Hülse aus einer für Feuchtigkeit undurchlässigen Cellophanfolie oder Polyproyplenfolie. Sie kann an ihrem rückwärtigen Ende bei 10a bereits dicht verschlossen sein, z.B. durch Heißversiegelung. Anschließend wird das vordere, gerundete Ende verschlossen, vorzugsweise ebenfalls durch Heißversiegelung. Im Rahmen der Erfindung sollte das für die Folienhülse 10 verwendete Folienmaterial sich relativ reckarm verhalten, d.h. zumindest in Umfangsrichtung ein geringes Dehnverhalten bei Zugbelastung aufweisen.

Um den Vorformling V vollständig mit Folienmaterial zu umschließen, ist die Folienhülse 10, selbst bei bereits vorab verschlossenem rückwärtigen Ende 10a, länger als der Vorformling V.

Der Durchmesser der vorgeformten zylindrischen Folienhülse 10 ist nur geringfügig größer, als der Durchmesser des entlang der zylindrischen Führungshülse 6 geschobenem Vorformlings V, um auf diese Weise dem runden Vorformling 1 ein ungehindertes Hineinschieben in die zylindrisch vorgeformte Folienhülse 10 zu ermöglichen.

Sodann wird, wie in Fig. 1 durch Pfeil 11 symbolisiert, der Vorformling V einschließlich der ihn auf ganzer Länge umgebenden Folienhülse 10 in eine dritte Verformungsstufe, die Pressstufe 15, überführt. In der dritten Verformungsstufe wird der Vorformling V zunächst in Fluchtung zu einer Presskammer 16 positioniert. Mittels eines axial in Richtung zu der Presskammer hin beweglichen Stößels 17 gelangt der Vorformling V sodann in die Presskammer 16 hinein, welche beim Ausführungsbeispiel zu zwei Seiten hin durch Preß- oder Druckplatten 20a, 20b begrenzt wird. Diese sind relativ zueinander beweglich, wofür es bereits ausreicht, wenn eine der beiden Preß- oder Druckplatten zu der anderen hin beweglich ist.

Wie Figur 2 erkennen lässt, verfügt jede Druckplatte 20a, 20b über eine Preß- oder Druckfläche 21, welche leicht muldenförmig gestaltet ist. Mittig zwischen den Druckflächen 21 wird der zu diesem Zeitpunkt noch im Wesentlichen zylindrische Vorformling V positioniert. Anschließend werden, wie durch die Druckpfeile 22 symbolisiert, die Druckplatten 20a, 20b einzeln oder beide aufeinander zu gefahren, wodurch der dazwischen angeordnete Vorformling V erneut verformt wird, und zwar entsprechend der Kontur der muldenförmigen Druckflächen 21. Beim dargestellten Beispiel verformt sich der zunächst kreiszylindrische Querschnitt des Vorformlings V zu einem eher ovalen Querschnitt des dann fertigen Tampons T, wie er in Figur 3 im Querschnitt dargestellt ist.

Aber auch andere, zu unrunden Querschnitten führende Verformungen sind möglich. Anstelle zweier Druckplatten 20a,20b kann auch mit drei oder mehr Druckplatten gearbeitet werden. Die Verformung in der letzten Pressstufe muss auch nicht zu einem leicht ovalen Querschnitt führen. Abhängig von der Gestalt der Druckflächen 21 ist auch eine Verformung zu z.B. einem elliptischen Querschnitt oder zu anderen, unrunden Querschnitten möglich.

In die Druckplatten 20a, 20b können Heizelemente integriert sein, um die Druckflächen 21, während diese sich schließen, zu beheizen.

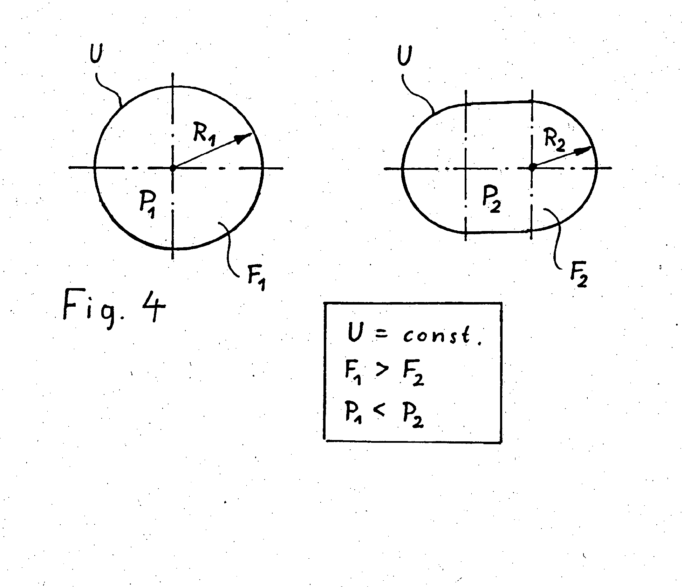

Figur 4 zeigt eine geometrische Herleitung der sich in der letzten Verformungsstufe einstellenden, zusätzlichen Verdichtung des Tampons. Da das für die Folie der Folienhülse verwendete Folienmaterial relativ reckarm ist, bleibt der Umfang U der Folienhülse und damit des Tampons vor und nach der letzten Pressstufe gleich. Dies führt aber zugleich zu einer Verringerung der Querschnittsfläche von F₁ zu F₂. Gleichzeitig verringert sich der Radius R₁ des Kreises auf den kleineren Bezugsradius R₂ eines Ovals. Da das von der Folienhülse eingeschlossene Fasermaterial des Tampons nicht anderweitig ausweichen kann, kommt es zu einer zusätzlichen Verdichtung des Fasermaterials mit Erhöhung des Innendrucks von P₁ auf P₂ bei der Überführung von einem Kreisquerschnitt auf den ovalen Querschnitt.

### Bezugszeichenliste

- 1: Presswerkzeug
- 1a: Pressbacke
- 1b: Pressschrieide
- 4: Formwerkzeug
- 4a: Eintrittsdurchmesser
- 4b: Austrittsdurchmesser
- 6: Zylindrische Führungshülse
- 7: Schräge
- 10: Folienhülse
- 10a: Verschlossenes Ende der Folienhülse
- 11: Pfeil
- 15: letzte Verformungsstufe
- 16: Presskammer
- 17: Stößel
- 20a: Druckplatte
- 20b: Druckplatte
- 21: Druckfläche
- 22: Druckpfeil
- F₁: Fläche
- F₂: Fläche
- R₁: Radius
- R₂: Radius
- T: (fertiger) Tampon
- U: Umfang
- V: Vorformling

## Patentansprüche

1. Verfahren zum Herstellen eines in einer Schutzhülle konfektionierten Tampons (T) aus einem durch einen Faservliesstreifen gebildeten Vorformling (V), welcher in getrennten Verformungsstufen durch von außen auf den Vorformling (V) einwirkende Druckkräfte zu dem Tampon (T) verformt, und welcher zur Einzelkonfektionierung mit einer den Tampon (T) fest umschließenden Folienhülse (10) als Schutzhülle versehen wird **dadurch gekennzeichnet, dass**
- die Folienhülse (10) aus einer für Feuchtigkeit undurchlässigen, unperforierten und zylindrisch glatt vorgeformten Cellophan- oder Polypropylen-Folie, die zumindest die Mantelfläche des Vorformlings (V) umschließt, hergestellt wird;
- der im Wesentlichen zylindrische Vorformling (V) noch vor der letzten Verformungsstufe (15) in die Folienhülse (10) hineingeschoben wird, und
- dass der Vorformling (V) einschließlich der ihn umschließenden Folienhülse (10) anschließend der letzten Verformungsstufe (15) zugeführt und dort zwischen mindestens zwei relativ zueinander beweglichen Druckbacken (20a, 20b) bis zum Erreichen einer nicht zylindrischen Endgestalt verformt, wird und
- der Tampon (T) durch dichtes Verschließen der Enden der Folienhülse (10) einzeln verpackt wird.

2. Verfahren nach Anspruch 1, **dadurch gekennzeichnet, dass** der Vorformling zwischen den Druckbacken (20a, 20b) zu einem länglichen Querschnitt verformt wird.

3. Verfahren nach Anspruch 1 oder Anspruch 2, dass die Druckbacken (20a, 20b) beheizt werden.

4. Verfahren nach Anspruch 1, 2 oder Anspruch 3, **dadurch gekennzeichnet, dass** die beiden Enden der Folienhülse (10) verschlossen werden, bevor der Vorformling (V) einschließlich der ihn vollständig umschließenden Folienhülse (10) der letzten Verformungsstufe (15) zugeführt wird.

5. Verfahren nach Anspruch 1 ,2 oder Anspruch 3, **dadurch gekennzeichnet, dass** die beiden Enden der Folienhülse (10) erst verschlossen werden, nachdem der Vorformling (V) zwischen den Druckbacken (20a, 20b) zu seiner Endgestalt verformt wurde.

6. Verfahren nach einem der vorangehenden Ansprüche, **gekennzeichnet durch** den Einsatz einer Folienhülse (10) aus einem reckarmen Folienmaterial auf Basis von Cellophan oder Polypropylen.

## Claims

1. Method of producing a tampon (T) packaged in a protective sleeve, which is made from a preform (V) provided in the form of a strip of fibrous non-woven material which is shaped to form the tampon (T) in separate forming stages by pressure forces acting on the preform (V) from outside, and which is individually packaged by providing it with a film sleeve (10) tightly enclosing the tampon (T) serving as a protective sleeve, **characterized in that**
- the film sleeve (10) is made from a cylindrically smooth, non-profiled cellophane film or polypropylene film that is impervious to moisture enclosing at least the external surface of the preform (V);
- the substantially cylindrical preform (V) is placed in the film sleeve (10) already before the final forming stage (15),
- and that the preform (V) including the film sleeve (10) surrounding it is then fed to the final forming stage (15), where it is formed between at least two pressing jaws (20a, 20b) which can be displaced relative to one another until it has assumed its final non-cylindrical final shape and
- the tampon (T) is individually packaged by tightly closing the ends of the film sleeve (10).

2. Method as claimed in claim 1, **characterized in that** the preform is formed to an elongate cross-section between the pressing jaws (20a, 20b).

3. Method as claimed in claim 1 or claim 2, **characterized in that** the pressing jaws (20a, 20b) are heated.

4. Method as claimed in claim 1, 2 or claim 3, **characterized in that** the two ends of the film sleeve (10) are closed before the preform (V) including the film sleeve (10) completely surrounding it is fed to the final forming stage (15).

5. Method as claimed in claim 1, 2 or claim 3, **characterized in that** the two ends of the film sleeve (10) are not closed until after the preform (V) has been formed to its final shape between the pressing jaws (20a, 20b).

6. Method as claimed in one of the preceding claims, **characterized by** the use of a film sleeve (10) made from a film material with a base of cellophane or polypropylene having a low capacity to stretch.

## Revendications

1. Procédé de fabrication d'un tampon (T) confectionné dans une gaine de protection, le tampon étant formé à partir d'une ébauche (V) constitué d'une bande en non-tissé, qui est formée en un tampon (T) en différentes étapes de déformation séparées par des efforts de pression agissant de l'extérieur sur l'ébauche (V), et qui est pourvue, à titre de confection individuelle, d'un manchon en film souple (10) formant une gaine de protection entourant fermement le tampon (T), **caractérisé en ce que**
- le manchon en film souple (10) est fabriqué à partir d'un film imperméable à l'humidité, non perforé et préformé en un cylindre lisse en cellophane ou polypropylène enfermant au moins la surface d'enveloppe de l'ébauche (V),
- l'ébauche (V) essentiellement cylindrique est introduite encore avant la dernière étape de déformation (15) dans le manchon en film souple (10), et
- que l'ébauche (V) avec le manchon en film souple (10) qui l'entoure est emmenée ensuite à la dernière étape de déformation (15) et y est déformée entre au moins deux contre-fers (20a, 20b) jusqu'à obtention d'une forme finale non cylindrique et
- le tampon (T) est conditionné individuellement par fermeture étanche des extrémités du manchon en film souple (10).

2. Procédé selon la revendication 1, **caractérisé en ce que** l'ébauche est déformée entre les contre-fers (20a, 20b) à une section transversale allongée.

3. Procédé selon la revendication 1 ou la revendication 2, **caractérisé en ce que** les contre-fers (20a, 20b) sont chauffés.

4. Procédé selon la revendication 1, 2 ou la revendication 3, **caractérisé en ce que** les deux extrémités du manchon en film souple (10) sont fermées avant que l'ébauche (V) avec le manchon en film souple (10) qui l'entoure entièrement, soit emmenée à la dernière étape de déformation (15).

5. Procédé selon la revendication 1, 2 ou la revendication 3, **caractérisé en ce que** les deux extrémités du manchon en film souple (10) sont fermées seulement après que l'ébauche (V) ait été déformée entre les contre-fers (20a, 20b) à sa forme finale.

6. Procédé selon l'une quelconque des revendications précédentes, **caractérisé par** l'utilisation d'un manchon en film souple (10) en un matériau en film souple à allongement très faible sur la base de cellophane ou de polypropylène.
